# EUROPÄISCHE PATENTANMELDUNG

(11) **EP 1 726 289 A2**
(43) Veröffentlichungstag der Anmeldung: **29.11.2006**
(21) Anmeldenummer: 05019088.3
(22) Anmeldetag: 02.09.2005
(51) Int. Cl.: A61K 8/19, A61K 8/67, A61Q 5/10

(54) **Reduktionsmittel-haltiges Mittel zum Färben von keratinhaltigen Fasern**

(30) Priorität: 28.10.2004 DE 102004052480
(71) Anmelder: Henkel Kommanditgesellschaft auf Aktien, 40589 Düsseldorf (DE)
(72) Erfinder: Höffkes, Horst, 40595 Düsseldorf (DE); Meinigke, Bernd, 51381 Leverkusen (DE); Bergmann, Bettina, 40667 Meerbusch (DE); Günther, Katja, 40721 Hilden (DE); Kleen, Astrid, 22763 Hamburg (DE)

(57) **Zusammenfassung**

Für brillante und intensive Farben enthalten Mittel zum Färben von Keratinfasern, insbesondere dem menschlichen Haar, mindestens ein Oxidationsfarbstoffvorprodukt und/oder mindestens einen direktziehenden Farbstoff sowie - bezogen auf das Gewicht des Mittels - mindestens 1 Gew.-% mindestens eines wasserlöslichen Reduktionsmittels.

## Beschreibung

Die Erfindung betrifft Mittel zum Färben von keratinhaltigen Fasern, insbesondere menschlicher Haare.

Für das Färben von keratinhaltigen Fasern kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridin-derivate verwendet. Als Kupplersubstanzen eignen sich insbesondere α-Naphthol, 1,5-, 2,7- und 1,7-Dihydroxynaphthalin. 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Schließlich hat ein weiteres Färbeverfahren große Beachtung gefunden. Bei diesem Verfahren werden Vorstufen des natürlichen Haarfarbstoffes Melanin auf das Haar aufgebracht; diese bilden dann im Rahmen oxidativer Prozesse im Haar naturanaloge Farbstoffe aus. Ein solches Verfahren verwendet beispielsweise 5,6-Dihydroxyindolin als Farbstoffvorprodukt. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden Auch hier können dann Probleme hinsichtlich der Echtheit der Färbungen auftreten.

Eine weitere Möglichkeit keratinhaltige Fasern zu färben, bietet die Verwendung von Färbemitteln, die eine Kombination aus Komponente
A Verbindungen, die eine reaktive Carbonylgruppe enthalten mit Komponente
B Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden
enthalten. Die entsprechende Färbemethode wird im Folgenden Oxofärbung genannt. Die resultierenden Färbungen besitzen teilweise Farbechtheiten auf der keratinhaltigen Faser, die mit denen der Oxidationsfärbung vergleichbar sind. Das mit der schonenden Oxofärbung erzielbare Nuancenspektrum ist sehr breit und die erhaltene Färbung weist oftmals eine akzeptable Brillanz und Farbtiefe auf. Die vorgenannten Komponenten A und B sind im allgemeinen selbst keine Farbstoffe, und eignen sich daher jede für sich genommen allein nicht zur Färbung keratinhaltiger Fasern. In Kombination bilden sie in einem nichtoxidativen Prozess Farbstoffe aus. Unter Verbindungen der Komponente B können allerdings auch entsprechende Oxidationsfarbstoffvorprodukte vom Entwickler- und/oder Kupplertyp mit oder ohne Einsatz eines Oxidationsmittels Verwendung finden. Somit läßt sich die Methode der Oxofärbung ohne weiteres mit dem oxidativen Färbesystem kombinieren.

Es hat nicht an Anstrengungen gefehlt, Färbungen keratinhaltiger Fasern zu verbessern. Insbesondere bei dem am häufigsten angewendeten Verfahren der oxidativen Haarfärbung besteht nach wie vor das Bedürfnis, die Applikationszeiten ohne Beeinträchtigung der Farbintensität und Dauerhaftigkeit der Färbung zu verringern und/oder auf den Einsatz unangenehm riechender Komponenten verzichten zu können.

Aufgabe der vorliegenden Erfindung war es daher, Färbemittel für Keratinfasern bereitzustellen, welche intensive und langanhaltende Färbungen liefern und weniger geruchsbelästigend für den Anwender sind.

Es wurde nun gefunden, daß der Einsatz von wasserlöslichen Reduktionsmitteln, die in der Lage sind, das Wasserstoffperoxid von Oxidationshaarfärbemitteln zu reduzieren, zu einer Temperaturerhöhung bei der Anwendung führt, welche genutzt werden kann, um den Gehalt an intensiv riechenden Komponenten, insbesondere Ammoniak, zu verringern.

Gegenstand der vorliegenden Erfindung ist in einer ersten Ausführungsform ein Mittel zum Färben von Keratinfasern, insbesondere dem menschlichen Haar, enthaltend mindestens ein Oxidationsfarbstoffvorprodukt und/oder mindestens einen direktziehenden Farbstoff, wobei das Mittel bezogen auf sein Gewicht mindestens 1 Gew.-% mindestens eines wasserlöslichen Reduktionsmittels enthält.

"Wasserlöslich" im Sinne der vorliegenden Anmeldung ist ein Reduktionsmittel wenn sich bei 20°C mindestens 50 Gramm des Reduktionsmittels in einem Liter destilliertem Wasser rückstandsfrei lösen. Bevorzugte erfindungsgemäß einsetzbare Reduktionsmittel weisen bei 20°C in destilliertem Wasser Löslichkeiten von mindestens 100 g/l, vorzugsweise von mindestens 150 g/l und insbesondere von mindestens 200 g/l auf.

"Reduktionsmittel" im Sinne der vorliegenden Erfindung sind Stoffe, die von Wasserstoffperoxid oxidiert werden können, d.h. als Reduktionsmittel für Wasserstoffperoxid geeignet sind. Besonders bevorzugte erfindungsgemäße Mittel sind dadurch gekennzeichnet, daß das/die Reduktionsmittel bei 25°C; 10⁵ Pa Druck, pH 7, in 1 molarer wäßriger Lösung ein Redoxpotential < -0,42 V aufweisen.

Aus der Vielzahl der möglichen Reduktionsmittel haben sich insbesondere Schwefelverbindungen als geeignet erwiesen, wobei unter diesen die Sulfite bzw. Hydrogensulfite und die Dithionite bevorzugt sind. Weitere geeignete Reduktionsmittel sind beispielsweise die Thiosulfate oder organische Reduktionsmittel wie Ascorbinsäure. Besonders bevorzugte erfindungsgemäße Mittel sind dadurch gekennzeichnet, daß das/die Reduktionsmittel ausgewählt ist/sind aus der Gruppe Natriumsulfit/Natriummetabisulfit, Natriumdithionit, Ascorbinsäure oder deren Salzen.

Das bzw. die Reduktionsmittel können in den erfindungsgemäßen Mitteln in variierenden Mengen enthalten sein, je nachdem, wie stark die Effekte der Temperaturerhöhung und Farbintensivierung gewünscht werden. Üblicherweise werden Mengen von bis 25 Gew.-%, jeweils bezogen auf das gesamte Mittel, eingesetzt, wobei bevorzugte erfindungsgemäße Mittel dadurch gekennzeichnet sind, daß sie das/die Reduktionsmittel in Mengen von 1,1 bis 15 Gew.-%, vorzugsweise von 1,5 bis 10 Gew.-% und insbesondere von 2,0 bis 7,5 Gew.-%, jeweils bezogen auf das Mittel, enthalten.

Durch den Einsatz der Reduktionsmittel werden die Applikationstemperaturen der Anwendungszusammensetzungen erhöht, vorzugsweise auf Werte im Bereich der Kopfhauttemperatur. Durch diese Temperaturerhöhung wird die Leistung der Mittel gesteigert, so daß insbesondere brillantere und intensivere Farben erzielt werden. Zudem ist es möglich, die höhere Leistung der erfindungsgemäßen Mittel dahingehend zu nutzen, andere leistungssteigernde aber vom Verbraucher als unangenehm empfundene Zusätze zu reduzieren oder ganz auf sie zu verzichten. Ein wichtiges Beispiel für solche Zusätze ist Ammoniak, der die Leistung von Haarfärbemitteln steigert, aber wegen seines stechenden Geruches vom Verbraucher als unangenehm empfunden wird. Bevorzugte erfindungsgemäße Mittel enthalten daher geringere Mengen an Ammoniak als vergleichbare Produkte des Standes der Technik.

Erfindungsgemäß weiter bevorzugte Mittel sind dadurch gekennzeichnet, daß sie frei von Ammoniak sind.

Durch den Einsatz der Reduktionsmittel wird die Temperatur der erfindungsgemäßen Mittel bei der Anwendung erhöht, wobei die temperaturerhöhende Reaktion zu Beginn des Anwendungsprozesses stattfindet. Demnach kühlt sich das erfindungsgemäße Mittel bei der Anwendung wieder auf Umgebungstemperatur ab, sofern nicht eine weitere exotherme Reaktion stattfindet. Dies kann man bei den erfindungsgemäßen Mitteln dadurch erreichen, daß mindestens ein Anteil eines Reduktionsmittels so konfektioniert wird, daß das Reduktionsmittel zeitverzögert freigesetzt wird. Durch die zeitverzögerte Freisetzung findet die exotherme Reaktion ebenfalls zeitverzögert statt, so daß eine länger anhaltende Temperaturerhöhung gewährleistet ist.

Die Konfektionierung des bzw. der Reduktionsmittel(s) zu Ermöglichung einer "kontrollierten Freisetzung", neudeutsch gerne als ''controlled release" bezeichnet, kann beispielsweise durch Einarbeitung in eine langsam lösliche Matrix erfolgen. Die Matrix kann aber auch durch Inhaltsstoffe der erfindungsgemäßen Mittel langsam chemisch reagieren und dabei zersetzt werden. Auch Temperatureffekte sind zur Zerstörung der Matrix realisierbar. Anstelle einer Matrix, in die das bzw. die Reduktionsmittel eingebettet sind, können auch individuell beschichtete Reduktionsmittel-Partikel eingesetzt werden. Bei solchen Partikeln ist bezogen auf ein gegebenes Gewicht eine höhere Menge an Reduktionsmitteln realisierbar, da die Beschichtung weniger Raum und Masse einnimmt als eine Matrix, in der mehrere Reduktionsmittelpartikel suspendiert vorliegen.

Vorzugsweise werden daher beschichtete Reduktionsmittel eingesetzt. Die Materialien, mit denen die Reduktionsmittel beschichtet werden können, sind dabei vorzugsweise aus wasserlöslichen, höhermolekularen Stoffen ausgewählt, wobei aber auch niedermolekulare Salze eingesetzt werden können.

Vorzugsweise weisen die beschichteten Partikel eine Größe zwischen 100 und 5000 µm, besonders bevorzugt zwischen 200 und 4000 µm, weiter bevorzugt zwischen 250 und 3000 µm, noch weiter bevorzugt zwischen 300 und 2000 µm und insbesondere zwischen 400 und 1000 µm auf. Die Dicke der Beschichtung beträgt vorzugsweise 0,1 bis 100 µm, besonders bevorzugt 0,5 bis 70 µm und insbesondere 1 bis 50 µm, wobei Werte zwischen 2 und 35 µm noch weiter bevorzugt sind.

Die beschichteten Reduktionsmittel-Partikel können auch als "Mikrokapseln" in die erfindungsgemäßen Mittel eingearbeitet werden. Unter dem Begriff "Mikrokapsel" werden Aggregate verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Die mikroskopisch kleinen Kapseln, auch Nanokapseln genannt, lassen sich wie Pulver trocknen. Neben einkernigen Mikrokapseln sind auch mehrkernige Aggregate, auch Mikrosphären bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Bevorzugt sind einkernige Mikrokapseln mit einer kontinuierlichen Hülle. Die Hülle kann aus natürlichen, halbsynthetischen oder synthetischen Materialien bestehen. Natürliche Hüllmaterialien sind beispielsweise Gummi arabicum, Agar Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine. Gelatine, Albumin, Schellack, Polysaccaride, wie Stärke oder Dextran, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon. Besonders bevorzugte erfindungsgemäße Mittel sind dadurch gekennzeichnet, daß sie mindestens ein verkapseltes (beschichtetes) Reduktionsmittel enthalten, wobei bevorzugte Verkapselungs- bzw Beschichtungsmaterialien ausgewählt sind aus der Gruppe Gummi arabicum, Agar, Agarose, Maltodextrine, Alginsäure, Alginaten, Fetten, Fettsäuren, Cetylalkohol, Collagen, Chitosan, Lecithin, Gelatine, Albumin, Schellack, Polysaccariden, Cellulosen, Celluloseestern, Celluloseethern, Stärkeethern, Stärkeestern, Polyacrylaten, Polyamiden, Polyvinylalkoholen und Polyvinylpyrrolidon.

Die Mikrokapseln können im herstellungsbedingten Rahmen eine beliebige Form aufweisen, sie sind jedoch bevorzugt näherungsweise kugelförmig. Ihr Durchmesser entlang ihrer größten räumlichen Ausdehnung kann je nach den in ihrem Inneren enthaltenen Substanzen und der Anwendung zwischen 10 nm (visuell nicht als Kapsel erkennbar) und 10 mm liegen. Bevorzugt sind sichtbare Mikrokapseln mit einem Durchmesser im Bereich von 0.1 mm bis 7 mm, insbesondere von 0,4 mm bis 5 mm. Nicht mehr mit bloßem Auge wahrnehmbare Mikrokapseln haben vorzugsweise einen Durchmesser im Bereich von 20 bis 500 nm, vorzugsweise 50 bis 200 nm. Besonders bevorzugte erfindungsgemäße Mittel sind dadurch gekennzeichnet, daß die Mikrokapseln einen Durchmesser entlang ihrer größten räumlichen Ausdehnung von 0.01 bis 10.000 µm aufweisen.

Die Mikrokapseln sind nach im Stand der Technik bekannten Verfahren zugänglich, wobei der Koazervation und der Grenzflächenpolymerisation die größte Bedeutung zukommt. Als Mikrokapseln lassen sich sämtliche auf dem Markt angebotenen tensidstabilen Mikrokapseln einsetzen, beispielsweise die Handelsprodukte (in Klammern angegeben ist jeweils das Hüllmaterial) *Hallcrest Microcapsules* (Gelatine, Gummi Arabicum), *Coletica Thalaspheres* (maritimes Collagen), *Lipolec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide).

Die Freisetzung der Substanzen aus den Mikrokapseln erfolgt üblicherweise während der Anwendung der sie enthaltenden Zubereitungen durch Zerstörung der Hülle infolge mechanischer, thermischer, chemischer oder enzymatischer Einwirkung. Vorzugsweise erfolgt die Freisetzung bei den erfindungsgemäßen Mitteln durch mechanische Einwirkung, insbesondere durch mechanische Kräfte, denen die Mikrokapseln beim Dosieren, vor oder bei der Anwendung ausgesetzt werden.

Die erfindungsgemäßen Mittel enthalten vorzugsweise wenig oder gar keinen Ammoniak. Als Akalisierungsmittel können sie vorzugsweise organische Amine enthalten, wobei primäre Amine gegenüber sekundären oder tertiären Aminen bevorzugt sind. Eine besonders bevorzugte Klasse von Aminen sind die Alkanolamine, beispielsweise Mono-, Di- und Trimethanolamin, Mono-, Di- und Triethanolamin, Mono-, Di- und Tri(n-propanol)amin sowie Mono-, Di- und Triisopropanolamin. Besonders bevorzugte erfindungsgemäße Mittel sind dadurch gekennzeichnet, daß sie organische Amine, vorzugsweise Alkanolamine, weiter bevorzugt Ethanolamine und insbesondere Monoethanolamin, enthalten.

Die erfindungsgemäßen Mittel enthalten mindestens ein Oxidationsfarbstoffvorprodukt und/oder mindestens einen direktziehenden Farbstoff. Diese Substanzen werden nachstehend beschrieben.

Bevorzugte erfindungsgemäße Oxidationshaarfärbemittel sind dadurch gekennzeichnet, daß sie mindestens eine Kupplerkomponente und mindestens eine Entwicklerkomponente enthält, wobei bevorzugte Mittel dadurch gekennzeichnet sind daß sie die Kupplerkomponente(n) in einer Menge von 0,01 bis 20 Gew.-%, vorzugsweise von 0,5 bis 5 Gew.-%, und die Entwicklerkomponente(n) in einer Menge von 0,01 bis 20 Gew.-%, vorzugsweise von 0,5 bis 5 Gew.-%, jeweils bezogen auf das gesamte Oxidationsfärbemittel, enthalten.

Diese erfindungsgemäßen Mittel enthalten vorzugsweise mindestens eine Kupplerkomponente aus der Gruppe der m-Phenylendiaminderivate, Naphthole, Resorcin und Resorcinderivate, Pyrazolone, m-Aminophenole und substituierte Pyridin-derivate. Diese werden nachfolgend beschrieben.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2.6-Dimethyl-3-aminophenol, 3-Trifluoracetylamino-2-chlor-6-methylphenol, 5-Amino-4-chlor-2-methylphenol, 5-Amino-4-methoxy-2-methylphenol. 5-(2'-Hydroxyethyl)-amino-2-methylphenol, 3-(Diethylamino)-phenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)propan, 1-Methoxy-2-amino-4-(2'-hydroxyethylamino)-benzol, 1,3-Bis-(2',4'-diaminophenyl)propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether. 2-Methylresorcin, 5-Methylresorcin, 2.5-Dimethylresorcin. 2-Chlorresorcin. 4-Chlorresorcin, Pyrogallol und 1,2,4-Trihydroxybenzol.
- Pyridinderivate wie beispielsweise 2,6-Dihydroxypyridin, 2-Amino-3-hydroxypyridin, 2-Amino-5-chfor-3-hydroxypyridin. 3-Amino-2-methylamino-6-methoxypyridin. 2.6-Dihydroxy-3,4-dimethylpyridin, 2.6-Dihydroxy-4-methylpyridin, 2.6-Diaminopyridin. 2.3-Diamino-6-methoxypyridin und 3.5-Diamino-2.6-dimethoxypyridin.
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1.8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4.6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol,

Besonders bevorzugte Kupplerkomponenten sind 2-Amino-3-hydroxypyridin, 2-Amino-3-hydroxy-5-chlorpyridin, 3-Amino-2-methylamino-6-methoxy-pyridin, 3,5-Diamino-2.6-dimethoxypyridin, 2,6-Dihydroxy-3,4-dimethylpyridin, m-Phenylendiamin, 2,6-Bis-(2-hydroxyethylamino)-toluol, 3-Amino-2,4-dichlorphenol, 3-Amino-2-chlor-6-methyfphenol, 5-Amino-4-chlor-2-methylphenol, 5-(β-Hydroxyethyl)-amino-2-methylphenol, 5-Amino-2-methylphenol, 2-Methylresorcin, 2-(2',4'-Diaminophenoxy)ethanoi, 1,3-Bis-(2',4'-diaminophenoxy)propan, Resorcin, 4-Chlorresorcin, Resorcinmonomethylether, m-Aminophenol, 1,7-, 2,7- und 1,5-Dihydroxynaphthalin sowie 4-Hydroxyindol und 6-Hydroxyindol.

Weitere bevorzugt einsetzbare Kupplerkomponenten sind 2-({3-[(2-Hydroxyethyl)amino]-4-methoxy-5-methylphenyl}amino)ethanol 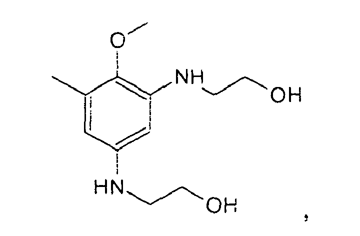 2-({3-[(2-Hydroxyethyl)amino]-2-methoxy-5-methy)phenyl}amino)ethanol 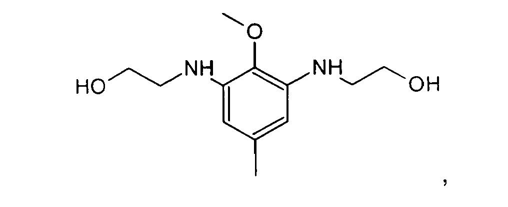 3-Amino-4-(2-methoxyethoxy)-5-methylphenylamin 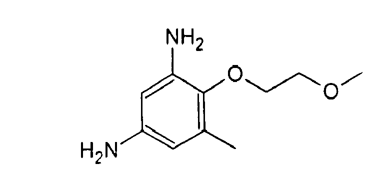 und
2-[(3-Morpholin-4-ylphenyl)amino]ethanol dihydrochlorid 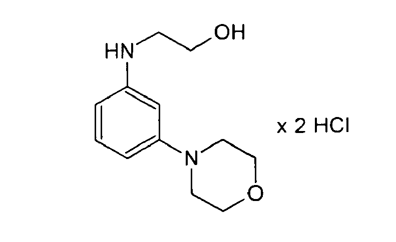 Zusammenfassend sind erfindungsgemäße Oxidationsfärbemittel bevorzugt, bei denen die Kupplerkomponente ausgewählt ist aus Resorcin, 3-Amino-2-methylamino-6-methoxypyridin, 3-Amino-6-methylphenol, 3-Amino-2-hydroxypyridin, 1,3-Bis-(2,4-diaminophenoxy)propan, 2,7-Dihydroxynaphthalin, 2-Methylresorcin, 5-Methyl-resorcin. 2,5-Dimethylresorcin und 4-Chlorresorcin 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methyl-resorcin, 5-Methylresorcin, 2,5-Dimethylresorcin, 2,6-Dihydroxy-3,4-dimethylpyridin, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol. 3-Amino-4-(2-methoxyethoxy)-5-methylphenylamin und 2-[(3-Morpholin-4-ylphenyl)aminoJethanol dihydrochlorid.

Die erfindungsgemäßen Oxidationsfärbemittel enthalten die Kupplerkomponente(n) bevorzugt in einer Menge von 0,01 bis 20 Gew.-%, vorzugsweise von 0,5 bis 5 Gew.-%, und die Entwicklerkomponente(n) in einer Menge von 0,01 bis 20 Gew.-%, vorzugsweise von 0,5 bis 5 Gew.-%, jeweils bezogen auf das gesamte Oxidationsfärbemittel.

Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1:0,5 bis 1:3, insbesondere 1:1 bis 1:2, enthalten sein können.

Allein mit einer Entwicklerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt. Es besteht daher ständig Bedarf an neuen, verbesserten Farbstoffkombinationen. Daher können andere bevorzugte Oxidationshaarfärbemittel weitere Entwicklerkomponenten enthalten.

Als weitere Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Es kann erfindungsgemäß bevorzugt sein, als zusätzliche Entwicklerkomponente ein p-Phenylendiaminderivat oder eines seiner physiologisch verträglichen Salze einzusetzen. Besonders bevorzugt sind p-Phenylendiaminderivate der Formel (E1) 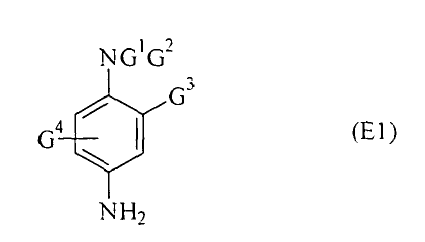 wobei
- G¹ steht für ein Wasserstoffatom, einen C₁-C₄-Alkylrest, einen C₁-C₄₋Monohydroxyalkylrest, einen C₂-C₄-Polyhydroxyalkylrest, einen (C₁-C₄)-Alkoxy-(C₁₋C₄)-alkylrest, einen 4'-Aminophenylrest oder einen C₁-C₄-Alkylrest, der mit einer stickstoffhaltigen Gruppe, einem Phenyl- oder einem 4'-Aminophenylrest substituiert ist,
- G² steht für ein Wasserstoffatom, einen C₁-C₄-Alkylrest, einen C₁-C₄₋Monohydroxyalkylrest, einen C₂-C₄-Polyhydroxyalkylrest, einen (C₁-C₄)-Alkoxy-(C₁₋C₄)-alkylrest oder einen C₁-C₄-Alkylrest, der mit einer stickstoffhaltigen Gruppe substituiert ist,
- G³ steht für ein Wasserstoffatom, ein Halogenatom, wie ein Chlor-, Brom-, lod- oder Fluoratom, einen C₁-C₄-Alkylrest, einen C₁-C₄-Monohydroxyalkylrest, einen C₂-C₄₋Polyhydroxyalkylrest, einen C₁-C₄-Hydroxyalkoxyrest, einen C₁-C₄₋Acetylaminoalkoxyrest, einen C₁-C₄-Mesylaminoalkoxyrest oder einen C₁-C₄₋Carbamoylaminoalkoxyrest,
- G⁴ steht für ein Wasserstoffatom, ein Halogenatom oder einen C₁-C₄-Alkylrest oder
- wenn G³ und G⁴ in ortho-Stellung zueinander stehen, können sie gemeinsam eine verbrückende α,ω-Alkylendioxogruppe, wie beispielsweise einen Ethylendioxygruppe bilden.

Beispiele für die als Substituenten im Rahmen dieser Anmeldung genannten C₁-C₄₋Alkylreste sind die Gruppen Methyl, Ethyl, Propyl, Isopropyl und Butyl. Ethyl und Methyl sind bevorzugte Alkylreste. Beispiele für bevorzugte C₂-C₄-Alkenylreste sind Vinyl und Allyl. Erfindungsgemäß bevorzugte C₁-C₄-Alkoxyreste sind beispielsweise eine Methoxy- oder eine Ethoxygruppe. Weiterhin können als bevorzugte Beispiele für eine C₁-C₄₋Monohydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Ein Beispiel für eine bevorzugte C₂-C₄₋Polyhydroxyalkylgruppe ist die α,β-Dihydroxyethylgruppe. Beispiele für Halogenatome sind erfindungsgemäß F-, Cl- oder Br-Atome. Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (II) sind insbesondere die Aminogruppen, C₁-C₄-Monoalkylaminogruppen, C₁-C₄-Dialkylaminogruppen, C₁-C₄₋Trialkylammoniumgruppen, C₁-C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-Hydroxyethyl)amino-2-chloranilin, 2-(β-Hydroxyethyl)-p-phenylendiamin, 2-Fluor-p-phenylendiamin. 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 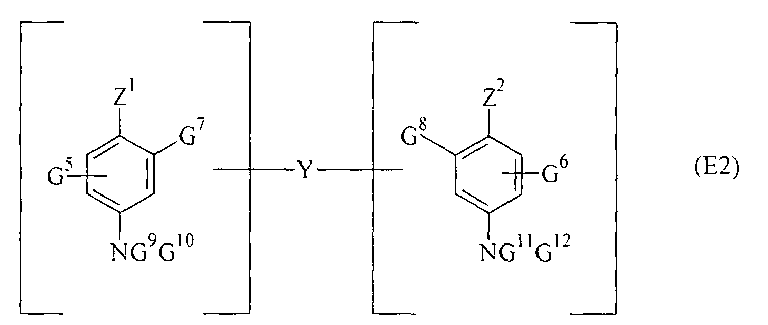 wobei:
- Z¹ und Z² stehen unabhängig voneinander für einen Hydroxyl- oder NH₂-Rest, das gegebenenfalls durch einen C₁-C₄-Alkylrest, durch einen C₁-C₄-Monohydroxyalkylrest und/oder durch eine Verbrückung Y substituiert ist oder das gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁-C₈₋Alkoxyreste substituiert sein kann, oder eine direkte Bindung,
- G⁵ und G⁶ stehen unabhängig voneinander für ein Wasserstoff- oder Halogenatom, einen C₁-C₄-Alkylrest, einen C₁-C₄-Monohydroxyalkylrest, einen C₂₋C₄₋Polyhydroxyalkylrest, einen C₁-C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁-C₄₋Alkylrest,
mit den Maßgaben daß
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminopheny)-1,3-diamino-propan-2-ol, N,N'-Bis-(β-hydroxyethyl)-N,N'-bis-(4'-aminophenyl)-ethylendiamin. N,N'-Bis-(4-aminophenyl)-tetra-methylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Bis-(ethyl)-N,N'-bis-(4'-amino-3'-methylphenyl)-ethylendiamin, Bis-(2-hydroxy-5-aminophenyl)-methan, 1,4-Bis-(4'-aminophenyl)-diaza-cycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1.3-diamino-propan-2-ol, Bis-(2-hydroxy-5-aminophenyl)-methan, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 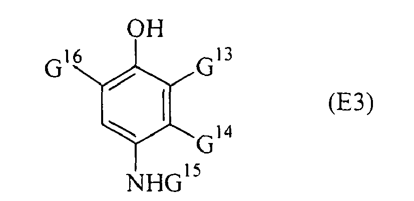 wobei
- G¹³ steht für ein Wasserstoffatom, ein Halogenatom, einen C₁-C₄-Alkylrest, einen C₁₋C₄-Monohydroxyalkylrest, einen C₂-C₄-Polyhydroxyalkylrest, einen (C₁-C₄)-Alkoxy-(C₁-C₄)-alkylrest, einen C₁-C₄-Aminoalkylrest, einen Hydroxy-(C₁-C₄)-alkylaminorest, einen C₁-C₄-Hydroxyalkoxyrest, einen C₁-C₄-Hydroxyalkyl-(C₁-bis C₄)-aminoalkylrest oder einen (Di-C₁-C₄-Alkylamino)-(C₁-C₄)-alkylrest, und
- G¹⁴ steht für ein Wasserstoff- oder Halogenatom, einen C₁-C₄-Alkylrest, einen C₁-C₄₋Hydroxyalkylrest, einen C₂-C₄-Polyhydroxyalkylrest, einen (C₁- C₄)-Alkoxy-(C₁-C₄)-alkylrest, einen C₁-C₄-Aminoalkylrest oder einen C₁- C₄-Cyanoalkylrest,
- G¹⁵ steht für Wasserstoff. einen C₁-C₄-Alkylrest, einen C₁-C₄-Hydroxyalkylrest, einen C₂-C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-Aminophenol, 4-Amino-3-methylphenol, 4-Amino-3-fluorphenol, 2-Hydroxy-methylamino-4-aminophenol, 4-Amino-3-hydroxymethylphenol, 4-Amino-2-(2-hydroxyethoxy)phenol, 4-Amino-2-methylphenol, 4-Amino-2-hydroxymethylphenol, 4-Amino-2-methoxymethylphenol, 4-Amino-2-aminomethylphenol, 4-Amino-2-(β-hydroxyethyl-aminomethyl)phenol, 4-Amino-2-fluorphenol, 4-Amino-2-chlorphenol, 2,6-Dichlor-4-aminophenol, 4-Amino-2-((diethylamino)methyl)phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol und 4-Amino-2-((diethylamino)methyl)phenol.

Ferner kann die zusätzliche Entwicklerkomponente ausgewählt sein aus o-Aminophenol und seinen Derivaten, wie beispielsweise 2-Amino-4-methylphenol oder 2-Amino-4-chlorphenol.

Weiterhin kann die zusätzliche Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen. Bevorzugt werden erfindungsgemäß Pyrimidin oder Pyrazolderivate.

Bevorzugte Pyrimidin-Derivate sind insbesondere die Verbindungen 2,4,5.6-Tetraaminopyrimidin. 4-Hydroxy-2.5.6-triaminopyrimidin. 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen 4,5-Diamino-1-methylpyrazol, 4,5-Diamino-1-(β-hydroxyethyl)-pyrazol, 3,4-Diaminopyrazol, 4,5-Diamino-1-(4'-chlorobenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2'-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4(β-hydroxyethyl)amino-1-methylpyrazol.

Bevorzugte Pyridin-Derivate sind insbesondere die Verbindungen 2,5-Diamino-pyridin, 2-(4-Methoxyphenyl)amino-3-amino-pyridin, 2,3-Diamino-6-methoxy-pyridin, 2-(ß-Methoxyethyl)amino-3-amino-6-methoxy-pyridin und 3,4-Diamino-pyridin.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 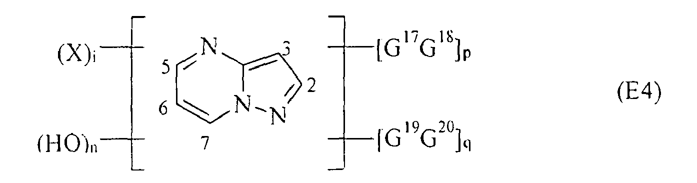 wobei.
- G¹⁷, G¹⁸, G¹⁹ und G²⁰ unabhängig voneinander stehen für ein Wasserstoffatom, einen C₁-C₄-Alkylrest einen Aryl-Rest, einen C₁-C₄-Hydroxyalkylrest, einen C₂-C₄₋Polyhydroxyalkylrest einen (C₁-C₄)-Alkoxy-(C₁-C₄)-alkylrest, einen C₁-C₄₋Aminoalkylrest, das gegebenenfalls durch einen Acetyl-Ureid- oder Sulfonyl-Rest geschützt sein kann, einen (C,-C₄)-Alkylamino-(C,-C₄)-alkylrest, einen Di-[(C₁-C₄)-alkyl]-(C₁-C₄)-aminoalkylrest, wobei die Dialkyl-Reste gegebenenfalls einen Kohlenstoffzyklus oder einen Heterozyklus mit 5 oder 6 Kettengliedern bilden, einen C₁-C₄-Hydroxyalkyl- oder einen Di-(C₁-C₄)-[Hydroxyalkyl]-(C₁-C₄)-aminoalkylrest,
- die X-Reste stehen unabhängig voneinander für ein Wasserstoffatom, einen C₁-C₄₋Alkylrest, einen Aryl-Rest, einen C₁-C₄-Hydroxyalkylrest, einen C₂-C₄₋Polyhydroxyalkylrest, einen C₁-C₄-Aminoalkylrest, einen (C₁-C₄)-Alkylamino-(C₁-C₄)-alkylrest, einen Di-[(C₁-C₄)alkyl]- (C₁-C₄)-aminoalkylrest, wobei die Dialkyl-Reste gegebenenfalls einen Kohlenstoffzyklus oder einen Heterozyklus mit 5 oder 6 Kettengliedern bilden, einen C₁-C₄-Hydroxyalkyl- oder einen Di-(C₁-C₄-hydroxyalkyl)-aminoalkylrest, einen Aminorest, einen C₁-C₄-Alkyl- oder Di-(C₁-C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, daß
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

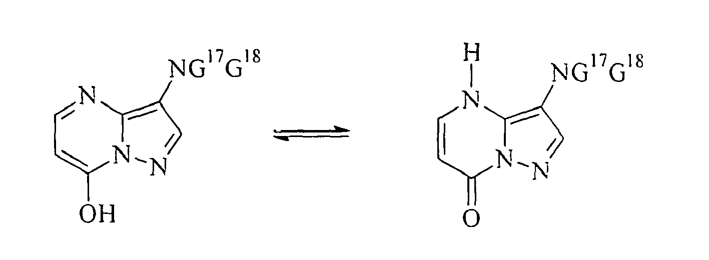

Unter den Pyrazol-[1,5-a]-pyrimidinen der obenstehenden Formel (E4) kann man insbesondere nennen:
- Pyrazol-[1,5-a]-pyrimidin-3,7-diamin; 2,5-Dimethyl pyrazol-[1,5-a]-pyrimidin-3,7-diamin;
- Pyrazol-(1,5-a]-pyrimidin-3,5-diamin;
- 2,7-Dimethyl pyrazol-[1,5-a]-pyrimidin-3,5-diamin;
- 3-Amino pyrazol-[1,5-a]-pyrimidin-7-ol;
- 3-Amino pyrazol-[1,5-a]-pyrimidin-5-ol;
- 2-(3-Amino pyrazol-[1,5-a]-pyrimidin-7-ylamino)-ethanol;
- 2-(7-Amino pyrazol-[1,5-a]-pyrimidin-3-ylamino)-ethanol;
- 2-[(3-Amino pyrazol-[1,5-a]-pyrimidin-7-yl)-(2-hydroxy-ethyl)-amino]-ethanol;
- 2-[(7-Amino pyrazol-[1,5-a]-pyrimidin-3-yl)-(2-hydroxy-ethyl)-amino]-ethanol;
- 5,6-Dimethyl pyrazol-[1,5-a]-pyrimidin-3,7-diamin;
- 2,6-Dimethyl pyrazol-[1,5-a]-pyrimidin-3,7-diamin;
- 2,5, N7, N7-Tetramethyl pyrazol-[1,5-a]-pyrimidin-3,7-diamin;
sowie ihre physiologisch verträglichen Salze und ihre tautomeren Formen, wenn ein tautomeres Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Erfindungsgemäß bevorzugte zusätzliche Entwicklerkomponenten sind Pyrimidinderivate, Pyrazolderivate, p-Aminophenolderivate und p-Phenylendiaminderivate.

Besonders bevorzugte zusätzliche Entwicklerkomponenten sind erfindungsgemäß p-Phenylendiamin, p-Toluylendiamin, 2-(β-Hydroxyethyl)-p-phenylendiamin, N,N-Bis-(β-hydroxyethyl)-p-phenylendiamin, N,N'-bis-(β-Hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan, 1,10-Bis-(2',5'-diaminophenyl)-1,4,7,10-tetraoxadecan, p-Aminophenol, 4-Amino-3-methylphenol, 4-Amino-3-fluorphenol, 4-Amino-2-aminomethylphenol 4-Amino-2-((diethylamino)methyl)phenol, o-Aminophenol, 2-Amino-4-methylphenol, 2-Amino-5-methylphenol, 2-Amino-4-chlorphenol, 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin, 2,5,6-Triaminopyrimidin, 1-(2'-Hydroxy-5'-aminobenzyl)-imidazolidin-2-on und 4,5-Diamino-1-(2'-hydroxyethyl)pyrazol.

Ganz besonders bevorzugte Entwicklerkomponenten sind erfindungsgemäß p-Toluylendiamin und 2,4,5,6-Tetraaminopyrimidin.

Eine weitere bevorzugt einsetzbare zusätzliche Entwicklerkomponente ist das N-(4-Amino-3-methylphenyl)-*N*-[3-(1*H*-imidazol-1-yl)propyl]amin trihydrochlorid):

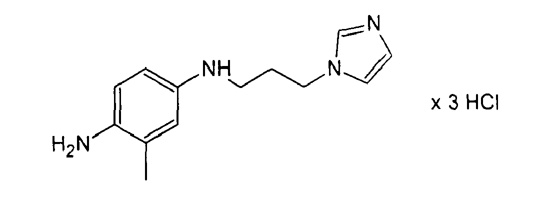

Zusammenfassend sind erfindungsgemäße Oxidationsfärbemittel bevorzugt, die eine weitere Entwicklerkomponente enthalten, die ausgewählt ist aus 3-Methyl-1,4-diaminobenzol, 1-(2'-Hydroxyethyl)-2,5-diaminobenzol, 2-(2,5-Diaminophenoxy)-ethanol, N,N-Bis(2'-Hydroxyethyl)-1,4-diaminobenzol, 3-Methyl-4-aminophenol und 2-Methylamino-4-aminophenol, p-Phenylendiamin, 2-(β-Hydroxyethyl)-p- phenylendiamin, N,N-Bis-(β-hydroxyethyl)-p-phenylendiamin, N,N'-bis-(β- Hydroxyethyl)-N,N'-bis- (4'-aminophenyl)-1,3-diamino-propan-2-ol, Bis-(2-hydroxy-5- aminophenyl)- methan, N,N'-Bis-(4'-aminophenyl)-1,4-diazacycloheptan, 1,10-Bis-(2',5'- diaminophenyl)-1,4,7,10-tetraoxadecan, p-Aminophenol, 4-Amino-3- methylphenol, 4- Amino-3-fluorphenol, 4-Amino-2-aminomethylphenol, 4- Amino-2- ((diethylamino)methyl)phenol, o-Aminophenol, 2-Amino-4- methylphenol, 2-Amino-5- methylphenol, 2-Amino-4-chlorphenol, 2,4,5,6-Tetraaminopyrimidin, 4-Hydroxy-2,5,6- triaminopyrimidin, 2-Hydroxy-4,5,6-triaminopyrimidin, 2-Dimethylamino-4,5,6- triaminopyrimidin, 2,4- Dihydroxy-5,6-diaminopyrimidin, 2,5,6-Triaminopyrimidin, 1-(2'- Hydroxy- 5'-aminobenzyl)-imidazolidin-2-on, 4,5-Diamino-1-(2'-hydroxyethyl) pyrazol und *N*-(4-Amino-3-methylphenyl)-*N*-[3-(1*H-*imidazol-1-yl)propyl]amin trihydrochlorid.

Weiterhin können die erfindungsgemäßen Mittel Vorstufen naturanaloger Farbstoffe enthalten. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (IIa), 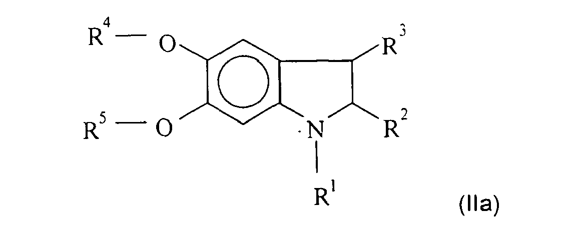 in der unabhängig voneinander
R¹ steht für Wasserstoff, eine C₁-C₄-Alkylgruppe, eine C₃-C₆-Cycloalkylgruppe, eine C₂-C₄-Alkenylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe.
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen.
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IIb), 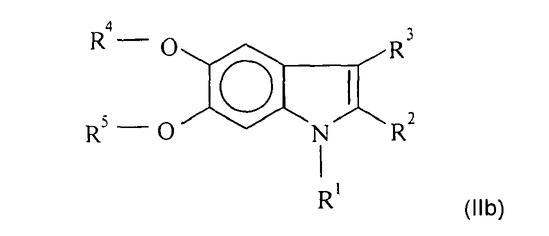 in der unabhängig voneinander
R¹ steht für Wasserstoff, eine C₁-C₄-Alkylgruppe, eine C₃-C₆-Cycloalkylgruppe, eine C₂-C₄-Alkenylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

Insbesondere bei der Verwendung von Farbstoff-Vorstufen vom Indolin- oder Indol-Typ hat es sich als vorteilhaft erwiesen, als Alkalisierungsmittel eine Aminosäure und/oder ein Oligopeptid einzusetzen.

In einer bevorzugten Ausführungsform enthalten die erfindungsgemäßen Haarfärbemittel zur weiteren Modifizierung der Farbnuancen neben den Oxidationsfarbstoffvorprodukten zusätzlich übliche direktziehende Farbstoffe.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1. HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1.4-Diamino-2-nitrobenzol, 2-Amino-4-nitrophenol, 1,4-Bis-(β-hydroxyethyl)amino-2-nitrobenzol. 3-Nitro-4-(β-hydroxyethyl)aminophenol. 2-(2'-Hydroxyethyl)amino-4,6-dinitrophenol. 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol. Entsprechende erfindungsgemäße Mittel, die dadurch gekennzeichnet sind, daß sie mindestens einen direktziehenden Farbstoff enthalten, der ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol, sind bevorzugt.

Ferner können die erfindungsgemäßen Mittel einen kationischen direktziehenden Farbstoff enthalten. Besonders bevorzugt sind dabei
- (a): kationische Triphenylmethanfarbstoffe, wie beispielsweise Basic Blue 7, Basic Blue 26, Basic Violet 2 und Basic Violet 14,
- (b): aromatischen Systeme, die mit einer quaternären Stickstoffgruppe substituiert sind, wie beispielsweise Basic Yellow 57, Basic Red 76. Basic Blue 99, Basic Brown 16 und Basic Brown 17, sowie
- (c): direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

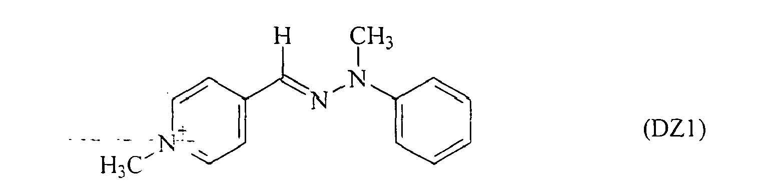

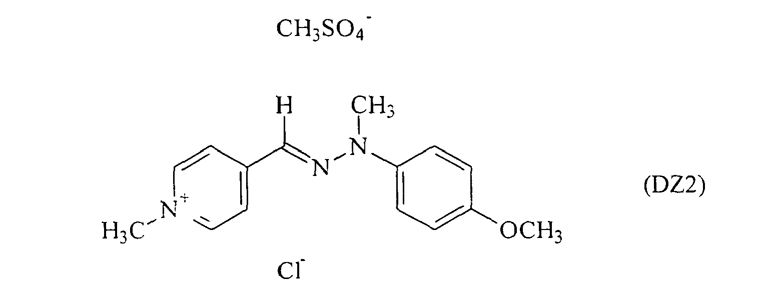

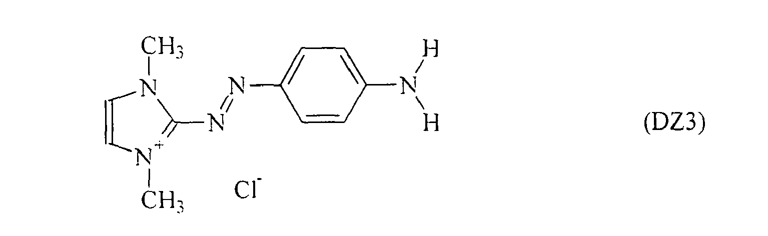

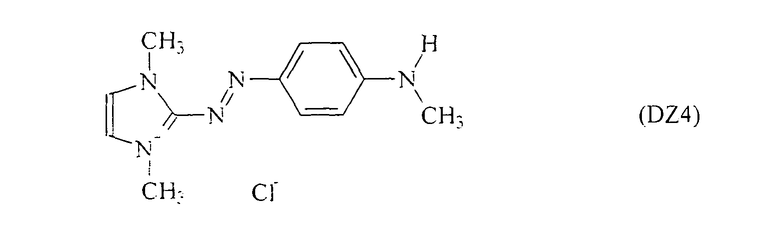

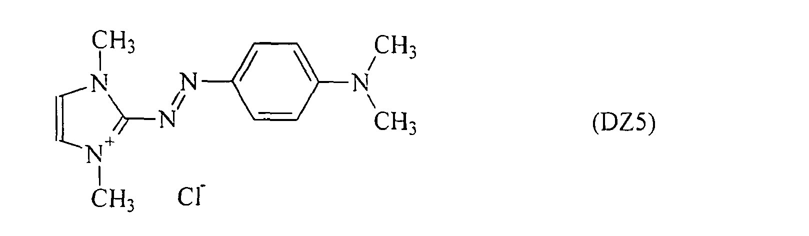

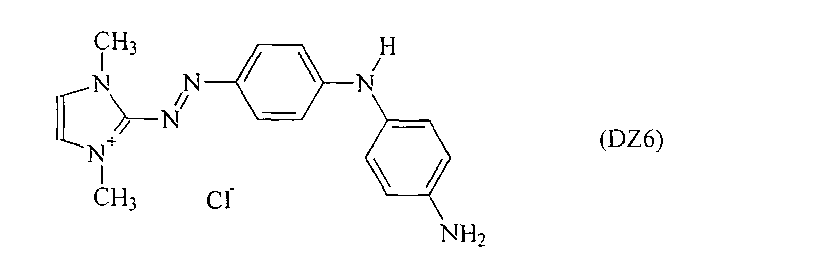

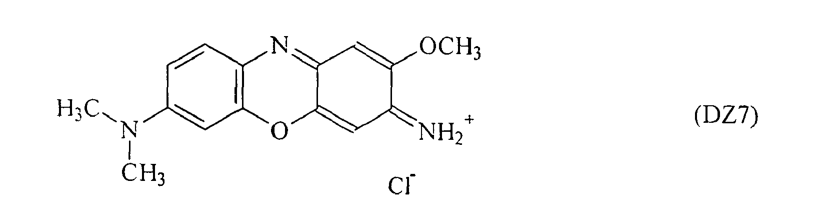

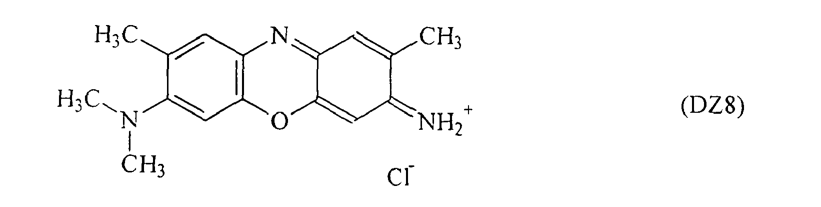

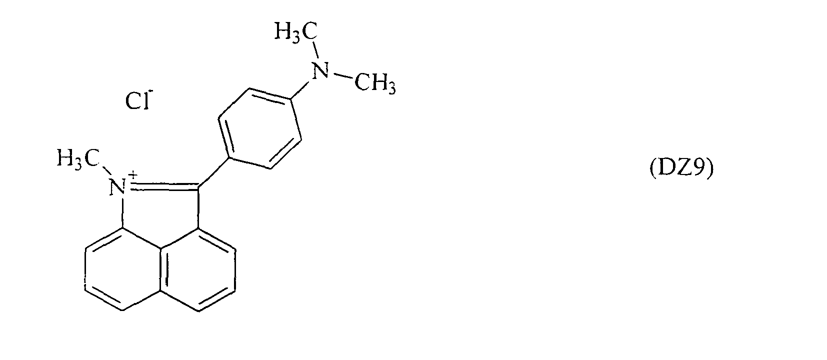

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c)

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel. Reformwaren und Körperpflegemittel e.V., Mannheim, Bezug genommen.

In einer weiteren Ausführungsform enthält das erfindungsgemäße Mittel zusätzlich eine Kombination aus Komponente
A Verbindungen, die eine reaktive Carbonylgruppe enthalten mit Komponente
B Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden.

Erfindungsgemäße Verbindungen mit einer reaktiven Carbonylgruppe (im Folgenden auch reaktive Carbonylverbindungen oder Komponente A genannt) besitzen mindestens eine Carbonylgruppe als reaktive Gruppe, welche mit den Verbindungen der Komponente B unter Ausbildung einer beide Komponenten verknüpfenden chemischen Bindung reagiert. Ferner sind erfindungsgemäß auch solche Verbindungen als Komponente A umfaßt, in denen die reaktive Carbonylgruppe derart derivatisiert bzw. maskiert ist, daß die Reaktivität des Kohlenstoffatoms der derivatisierten bzw. maskierten Carbonylgruppe gegenüber der Komponente B stets vorhanden ist. Diese Derivate sind bevorzugt Kondensationsverbindungen von reaktiven Carbonylverbindungen mit
a) Aminen und deren Derivate unter Bildung von Iminen oder Oximen als Kondensationsverbindung
b) von Alkoholen unter Bildung von Acetalen oder Ketalen als Kondensationsverbindung.

Die Komponente A wird bevorzugt ausgewählt aus der Gruppe, die gebildet wird aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxyacetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron,
Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxybenzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxybenzaldehyd, 4-Hydroxy-3,5-dimethyl-benzaldehyd, 3,5-Diethoxy-4-hydroxybenzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxybenzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,5-Dihydroxybenzaldehyd, 2,6-Dihydroxybenzaldehyd, 3,4-Dihydroxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd. 4-Hydroxy-1-naphthaldehyd, 4-Dimethylamino-1-naphthaldehyd, 2-Methoxy-zimtaldehyd, 4-Methoxy-zimtaldehyd, 4-Hydroxy-3-methoxy-zimtaldehyd, 3,5-Dimethoxy-4-hydroxy-zimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylaminozimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd, Piperonal, 2,3,6,7-Tetrahydro-1H,5H-benzo[ij]chinolizin-9-carboxaldehyd, 2,3,6,7-Tetrahydro-8-hydroxy-1H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd),
2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd, 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon,
Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzyliden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon,
5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal,
6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)hexa-3,5-dien-2-on, 6-(4-Pyrrolidinophenyl)hexa-3,5-dien-2-on,
5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzalde₋hyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd,
4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-imidazolium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'--Formylphenyl)-3,5-dimethylbenzothiazolium-benzolsulfonat, -p-toluolsulfonat, -methansulfonat, -perchlorat, -sulfat, -chlorid, -bromid, -iodid, -tetrachlorozinkat, -methylsulfat-, trifluormethansulfonat, -tetrafluoroborat,
Isatin, 1-Methyl-isatin, 1-Allyl-isatin, 1-Hydroxymethyl-isatin, 5-Chlor-isatin, 5-Methoxyisatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Als CH-acide werden im allgemeinen solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-Wasserstoff-Bindung bewirkt wird. Unter CH-acide Verbindungen fallen erfindungsgemäß auch Enamine, die durch alkalische Behandlung von quaternierten N-Heterozyklen mit einer in Konjugation zum quartären Stickstoff stehenden CH-aciden Alkylgruppe entstehen.

Die CH-aciden Verbindungen der Komponente B sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, 1,4-Dimethylchinolinium-iodid, 1,2-Dimethylchinolinium-iodid, Oxindol, 3-Indoxylacetat, 2-Cumaranon, 5-Hydroxy-2-cumaranon, 6-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat. Besonders bevorzugte CH-acide Verbindungen sind 1,2-Dihydro-1,3,4,6-tetramethyl-2-oxo-pyrimidiniumchlorid, 1,2-Dihydro-1,3-diethyl-4,6-dimethyl-2-oxo-pyrimidiniumchlorid, 1,2-Dihydro-1,3-dipropyl-4,6-dimethyl-2-oxo-pyrimidiniumchlorid, 1,2-Dihydro-1,3-bis(2-hydroxyethyl)-4,6-dimethyl-2-oxo-pyrimidiniumchlorid, 1,2-Dihydro-1,3,4,6-tetramethyl-2-oxo-pyrimidiniumhydrogen-sulfat, 1,2-Dihydro-1,3-diethyl-4,6-dimethyl-2-oxo-pyrimidiniumhydrogensulfat, 1,2-Dihydro-1,3-dipropyl-4,6-dimethyl-2-oxo-pyrimidiniumhydrogensulfat, 1,2-Dihydro-1,3-bis(2-hydroxyethyl)-4,6-dimethyl-2-oxo-pyrimidiniumhydrogensulfat, 1,2-Dihydro-1,3,4-trimethyl-2-oxo-pyrimidiniumchlorid, 1,2-Dihydro-1,3,4-trimethyl-2-oxo-pyrimidinium-hydrogensulfat, 1,2-Dihydro-1,3-diethyl-4-methyl-2-oxo-pyrimidiniumchlorid, 1,2-Dihydro-1,3-diethyl-4-methyl-2-oxo-pyrimidiniumhydrogensulfat, 1,2-Dihydro-1,3-dipropyl-4-methyl-2-oxo-pyrimidiniumchlorid, 1,2-Dihydro-1,3-dipropyl-4-methyl-2-oxo-pyrimidinium-hydrogensulfat, 1,2-Diyhdro-1,3-bis(2-hydroxyethyl)-4-methyl-2-oxo-pyrimidiniumchlorid sowie 1,2-Diyhdro-1,3-bis(2-hydroxyethyl)-4-methyl-2-oxo-pyrimidiniumhydrogensulfat.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3C-Atomen in der Alkanolgruppe,
- lineare Fettsäuren mit 10 bis 22C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18C-Atomen in der Acylgruppe,
- Sulfobernsteinsäuremono- und -dialkylester mit 8 bis 18C-Atomen in der Alkylgruppe und Sulfobernsteinsäuremono-alkylpolyoxyethylester mit 8 bis 18C-Atomen in der Alkylgruppe und 1 bis 6 Oxyethylgruppen,
- lineare Alkansulfonate mit 12 bis 18C-Atomen,
- lineare Alpha-Olefinsulfonate mit 12 bis 18C-Atomen,
- Alpha-Sulfofettsäuremethylester von Fettsäuren mit 12 bis 18C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.
   Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex® , Dehyquart® und Armocare® vertrieben. Die Produkte Armocare® VGH-70, ein N,N-Bis(2-Palmitoyloxy-ethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat® 100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die erfindungsgemäßen Färbemittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien,
enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel enthalten die Verbindungen gemäß Formel I bevorzugt in einem geeigneten wäßrigen, alkoholischen oder wäßrig-alkoholischen Träger. Zum Zwecke-der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Farbstoffvorprodukte in eine pulverförmige oder auch Tabletten-förmige Formulierung zu integrieren.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Die Gegenwart von Oxidationsfarbstoffvorprdukten ist demnach keine zwingende Voraussetzung für einen Einsatz von Oxidationsmitteln in den erfindungsgemäßen Mitteln. Als Oxidationsmittel kommen Persulfate, Chlorite und insbesondere Wasserstoffperoxid oder dessen Anlagerungsprodukte an Harnstoff, Melamin sowie Natriumborat in Frage.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, lodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Öxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Bei einer Anwendung von Oxidationsmitteln wird das eigentliche Färbemittel zweckmäßigerweise unmittelbar vor der Anwendung durch Mischung der Zubereitung des Oxidationsmittels mit der Zubereitung, enthaltend die Verbindungen der Formel 1 und gegebenenfalls Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40°C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann ein erfindungsgemäßes Mittel gegebenenfalls mit zusätzlichen Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Ein zweiter Gegenstand der vorliegenden Erfindung ist ein Verfahren zum Tönen und/oder Färben von Keratinfasern, insbesondere menschlichen Haaren, in dem
- gewünschtenfalls ein Vorbehandlungsmittel M1 auf die Faser aufgebracht wird, dann
- ein Tönungs- oder Färbemittel M2 auf der Faser zur Anwendung kommt, wobei gewünschtenfalls dem Mittel M2 vor der Anwendung ein weiteres Mittel M3 zugegeben wird,
- dieses Färbemittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird,
bei dem das Mittel M2 mindestens ein Oxidationsfarbstoffvorprodukt und/oder mindestens einen direktziehenden Farbstoff sowie - bezogen auf sein Gewicht - mindestens 1 Gew.-% mindestens eines wasserlöslichen Reduktionsmittels enthält, falls nicht das Vorbehandlungsmittel M1 mindestens 1 Gew.-% mindestens eines wasserlöslichen Reduktionsmittels enthält.

In erfindungsgemäß bevorzugten Verfahren wird dem Mittel M2 kurz vor der Anwendung ein Mittel M3, enthaltend mindestens ein Oxidationsmittel, zugegeben.

Besonders bevorzugte erfindungsgemäße Verfahren sind dabei dadurch gekennzeichnet, daß das Mittel M2 eine erfindungsgemäße Zubereitung ist.

Weitere Gegenstände der vorliegenden Erfindung sind die Verwendung von wasserlöslichen Reduktionsmitteln in Haarfärbemitteln und die Verwendung von wasserlöslichen Reduktionsmitteln zur Verbesserung der Färbeintensität von Haarfärbemitteln.

Bezüglich bevorzugter Ausführungsformen des erfindungsgemäßen Verfahrens und der erfindungsgemäßen Verwendung gilt mutatis mutandis das zu den erfindungsgemäßen Mitteln weiter oben Ausgeführte.

### Beispiele:

### 1a) Färbecreme (Gew.-%)

Creme-Grundlage (Teilmischung A)

| | |
|---|---|
| Hydrenol® D¹ | 8,50g |
| Lorol® techn.² | 2,00g |
| Eumulgin® B2³ | 0,75g |
| Texapon® NSO⁴ | 20,00g |
| Dehyton® K⁵ | 12,50g |
| Wasser | 30,00g |

- 1: C16-18-Fettalkohol (INCl-Bezeichnung: Cetearyl alcohol) (Cognis)
- 2: C12-18-Fettalkohol (INCl-Bezeichnung: Coconut alcohol) (Cognis)
- 3: Cetylstearylalkohol mit ca. 20 EO-Einheiten (INCI-Bezeichnung: Ceteareth-20) (Cognis)
- 4: Laurylethersulfat, Natriumsalz (ca. 27,5% Aktivsubstanz; INCI-Bezeichnung: Sodium Laureth Sulfate) (Cognis)
- 5: N,N-Dimethyl-N-(C8-18-kokosamidopropyl)ammoniumacetobetain (ca. 30% Aktivsubstanz; INCI-Bezeichnung: Aqua (Water), Cocamidopropyl Betaine) (Cognis)

Die Substanzen Hydrenol D, Lorol und Eumulgin B2 wurden bei 80°C aufgeschmolzen, mit dem 80°C heißem Wasser, enthaltend Texapon NSO und Dehyton K, vermischt und unter starkem Rühren emulgiert. Danach wurde die Emulsion unter schwachem Rühren abgekühlt.

Färbezubereitung (Teilmischung B1)
4-Aminophenol 0,27g (0,0025mol)
5-Amino-2-methylphenol 0,31g (0,0025mol)
Ammoniumhydroxid ad pH 7,0 bis 8,0
Wasser 20ml

Die Farbstoffvorprodukte wurden in 20ml Wasser gelöst und der pH-Wert der Lösung mit Ammoniumhydroxid eingestellt.

Die Farbstoffzubereitung (Teilmischung B1) wurden zu 25g der bei 80°C geschmolzen Cremegrundlage (Teilmischung A) gegeben und der pH-Wert gegebenenfalls mit einer wäßrigen HCl-Lösung oder mit Ammoniumhydroxid auf pH 7 eingestellt. Die Creme wurde mit Wasser auf 50g aufgefüllt und unter Rühren auf 30°C abgekühlt (Färbecreme).

### 1 b) Entwickleremulsion (Gew.-%)

| | |
|---|---|
| Lorol® C16* | 3,5 |
| Eumulgin® B2 | 1,0 |
| Disponil® FES 77lS** | 2,5 |
| Ammoniak-Lösung, 25%ig | 0,65 |
| Dipicolinsäure | 0,1 |
| Natriumpyrophosphat | 0,03 |
| Wasserstoffperoxidlösung, 50%ig | 12,0 |
| Wasser | ad 100 |

| | |
|---|---|
| * Cetylalkohol (INCI-Bezeichnung: Cetyl Alcohol) | |
| ** ethoxyliertes Fettalkoholsulfat (INCI-Bezeichnung: Sodium Coceth-30 Sulfate) (Cognis) | |

### 2. Färbung der keratinischen Fasern

Es wurden 40 ml Färbecreme und 50 ml Entwicklerdispersion miteinander vermischt. Zur Herstellung der erfindungsgemäßen Färbemittel wurden jeweils 2,5 g eines Reduktionsmittels zugegeben. Die Temperatur der so erhaltenen Färbecreme wurde direkt nach dem Mischen gemessen und die Färbecreme danach auf 5cm lange Strähnen standardisierten, zu 80% ergrauten, aber nicht besonders vorbehandelten Menschenhaares (Kerling Naturweiß) aufgetragen (Mengenverhältnis Farbstoffmischung : Haar betrug 4 : 1). Nach 30min Einwirkzeit wurde das Haar gespült, mit einem üblichen Haarwaschmittel ausgewaschen und anschließend getrocknet. Die Färbeergebnisse sind der nachstehenden Tabelle zu entnehmen.

| Färbecreme | Entwickler-dispersion | Reduktionsmittel | Temperatur | Farbe |
|---|---|---|---|---|
| 40 ml | 50ml | - | 26°C | dunkelbordeaux, Farbtiefe hellbraun |
| 40 ml | 50ml | + 2,5 g Natriumsulfit | 43°C | dunkler, Farbtiefe mittelbraun |
| 40 ml | 50ml | + 2,5 g Ascorbinsäure | 30,5°C | geringfügig heller, bräunlicher |
| 40 ml | 50ml | + 2,5 g Natriumdithionit | 59°C | dunkler, rötlicher, Farbtiefe mittelbraun |

Die Ergebnisse zeigen, daß sich die Intensität und Farbtiefe durch den Zusatz der Reduktionsmittel verbessern lassen.

## Patentansprüche

1. Mittel zum Färben von Keratinfasern, insbesondere dem menschlichen Haar, enthaltend mindestens ein Oxidationsfarbstoffvorprodukt und/oder mindestens einen direktziehenden Farbstoff, **dadurch gekennzeichnet, daß** das Mittel bezogen auf sein Gewicht mindestens 1 Gew.-% mindestens eines wasserlöslichen Reduktionsmittels enthält.

2. Mittel nach Anspruch 1, **dadurch gekennzeichnet, daß** das/die Reduktionsmittel bei 25°C; 10⁵ Pa Druck, pH 7, in 1 molarer wäßriger Lösung ein Redoxpotential < -0,42 V aufweisen.

3. Mittel nach einem der Ansprüche 1 oder 2, **dadurch gekennzeichnet, daß** das/die Reduktionsmittel ausgewählt ist/sind aus der Gruppe Natriumsulfit/Natriummetabisulfit, Natriumdithionit, Ascorbinsäure oder deren Salzen.

4. Mittel nach einem der Ansprüche 1 bis 3, **dadurch gekennzeichnet, daß** es das/die Reduktionsmittel in Mengen von 1,1 bis 15 Gew.-%, vorzugsweise von 1,5 bis 10 Gew.-% und insbesondere von 2,0 bis 7,5 Gew.-%, jeweils bezogen auf das Mittel, enthält.

5. Mittel nach einem der Ansprüche 1 bis 4, **dadurch gekennzeichnet, daß** es frei von Ammoniak ist.

6. Mittel nach einem der Ansprüche 1 bis 5, **dadurch gekennzeichnet, daß** es mindestens ein verkapseltes (beschichtetes) Reduktionsmittel enthält, wobei bevorzugte Verkapselungs- bzw. Beschichtungsmaterialien ausgewählt sind aus der Gruppe Gummi arabicum, Agar, Agarose, Maltodextrine, Alginsäure, Alginaten, Fetten, Fettsäuren, Cetylalkohol, Collagen, Chitosan, Lecithin, Gelatine, Albumin, Schellack, Polysaccariden, Cellulosen, Celluloseestern, Celluloseethern, Stärkeethern, Stärkeestern, Polyacrylaten, Polyamiden, Polyvinylalkoholen und Polyvinylpyrrolidon.

7. Mittel nach einem der Ansprüche 1 bis 6, **dadurch gekennzeichnet, daß** es organische Amine, vorzugsweise Alkanolamine, weiter bevorzugt Ethanolamine und insbesondere Monoethanolamin, enthält.

8. Mittel nach einem der Ansprüche 1 bis 7, **dadurch gekennzeichnet, daß** es mindestens eine Kupplerkomponente und mindestens eine Entwicklerkomponente enthält, wobei bevorzugte Mittel **dadurch gekennzeichnet sind, daß** sie die Kupplerkomponente(n) in einer Menge von 0,01 bis 20 Gew.-%, vorzugsweise von 0,5 bis 5 Gew.-%, und die Entwicklerkomponente(n) in einer Menge von 0,01 bis 20 Gew.-%, vorzugsweise von 0,5 bis 5 Gew.-%, jeweils bezogen auf das gesamte Oxidationsfärbemittel, enthalten.

9. Mittel nach Anspruch 8, **dadurch gekennzeichnet, daß** die Kupplerkomponente ausgewählt ist aus Resorcin, 3-Amino-2-methylamino-6-methoxypyridin, 3-Amino-6-methylphenol, 3-Amino-2-hydroxypyridin, 1,3-Bis-(2,4-diaminophenoxy)propan, 2,7-Dihydroxynaphthalin, 2-Methylresorcin, 5-Methyl-resorcin, 2,5-Dimethylresorcin und 4-Chlorresorcin 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin, 2,6-Dihydroxy-3,4-dimethylpyridin, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 3-Amino-4-(2-methoxyethoxy)-5-methylphenylamin und 2-[(3-Morpholin-4-ylphenyl)amino]ethanol dihydrochlorid.

10. Mittel nach einem der Ansprüche 8 oder 9, **dadurch gekennzeichnet, daß** die Entwicklerkomponente ausgewählt ist aus 3-Methyl-1,4-diaminobenzol, 1-(2'-Hydroxyethyl)-2,5-diaminobenzol, 2-(2,5-Diaminophenoxy)-ethanol, N,N-Bis(2'-Hydroxyethyl)-1,4-diaminobenzol, 3-Methyl-4-aminophenol und 2-Methylamino-4-aminophenol, p-Phenylendiamin, 2-(β-Hydroxyethyl)-p- phenylendiamin, N,N-Bis-(β-hydroxyethyl)-p-phenylendiamin, N,N'-bis-(β- Hydroxyethyl)-N,N'-bis- (4'-aminophenyl)-1,3-diamino-propan-2-ol, Bis-(2-hydroxy-5- aminophenyl)- methan, N,N'-Bis-(4'-aminophenyl)-1,4-diazacycloheptan, 1,10-Bis-(2',5'- diaminophenyl)-1,4,7,10-tetraoxadecan, p-Aminophenol, 4-Amino-3- methylphenol, 4- Amino-3-fluorphenol, 4-Amino-2-aminomethylphenol, 4- Amino-2-((diethylamino)methyl)phenol, o-Aminophenol, 2-Amino-4- methylphenol, 2-Amino-5- methylphenol, 2-Amino-4-chlorphenol, 2,4,5,6- Tetraaminopyrimidin, 4-Hydroxy-2,5,6- triaminopyrimidin, 2-Hydroxy-4,5,6- triaminopyrimidin, 2-Dimethylamino-4,5,6- triaminopyrimidin, 2,4- Dihydroxy-5,6-diaminopyrimidin, 2,5,6-Triaminopyrimidin, 1-(2'- Hydroxy- 5'-aminobenzyl)-imidazolidin-2-on, 4,5-Diamino-1-(2'-hydroxyethyl) pyrazol und *N*-(4-Amino-3-methylphenyl)-*N*-[3-(1*H*-imidazol-1-yl)propyl]amin trihydrochlorid.

11. Mittel nach einem der Ansprüche 1 bis 10, **dadurch gekennzeichnet, daß** es mindestens einen direktziehenden Farbstoff enthält, der ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

12. Verfahren zum Tönen und/oder Färben von Keratinfasern, insbesondere menschlichen Haaren, in dem
- gewünschtenfalls ein Vorbehandlungsmittel M1 auf die Faser aufgebracht wird, dann
- ein Tönungs- oder Färbemittel M2 auf der Faser zur Anwendung kommt, wobei gewünschtenfalls dem Mittel M2 vor der Anwendung ein weiteres Mittel M3 zugegeben wird,
- dieses Färbemittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird,
**dadurch gekennzeichnet, daß** das Mittel M2 mindestens ein Oxidationsfarbstoffvorprodukt und/oder mindestens einen direktziehenden Farbstoff sowie - bezogen auf sein Gewicht - mindestens 1 Gew.-% mindestens eines wasserlöslichen Reduktionsmittels enthält falls nicht das Vorbehandlungsmittel M1 mindestens 1 Gew.-% mindestens eines wasserlöslichen Reduktionsmittels enthält..

13. Verfahren nach Anspruch 12, **dadurch gekennzeichnet, daß** dem Mittel M2 kurz vor der Anwendung ein Mittel M3, enthaltend mindestens ein Oxidationsmittel, zugegeben wird.

14. Verfahren nach einem der Ansprüche 12 oder 13, **dadurch gekennzeichnet, daß** das Mittel M2 eine Zubereitung gemäß einem der Ansprüche 1 bis 11 ist.

15. Verwendung von wasserlöslichen Reduktionsmitteln in Haarfärbemitteln.

16. Verwendung von wasserlöslichen Reduktionsmitteln zur Verbesserung der Färbeintensität von Haarfärbemitteln.
